Europäisches Patentamt

European Patent Office

Office européen des brevets

(19)

(11) Publication number: **0 069 523**
**B1**

## EUROPEAN PATENT SPECIFICATION

(12)

(45) Date of publication of patent specification: **01.04.87**

(21) Application number: **82303388.1**

(22) Date of filing: **29.06.82**

(51) Int. Cl.[4]: **B 01 D 13/00, C 12 P 19/04, C 08 B 37/00**

(54) Concentration of aqueous pseudoplastic solutions by membrane ultrafiltration.

(30) Priority: **01.07.81 US 279402**

(43) Date of publication of application:
**12.01.83 Bulletin 83/02**

(45) Publication of the grant of the patent:
**01.04.87 Bulletin 87/14**

(84) Designated Contracting States:
**DE FR GB IT NL**

(56) References cited:
**EP-A-0 049 012**
**DE-A-2 737 989**
**US-A-3 856 569**
**US-A-4 299 825**

**CHEMIE INGENIEUR TECHNIK, vol. 45, no. 12, June 1973, pages 825-832, Weinheim, DE, H. STRATHMANN: "Untersuchungen zur Konzentrationsüberhöhung bei der Membranfiltration"**

**INDUSTRIAL WATER ENGINEERING, June/July 1971, pages 18-24, M.C. PORTER et al.: "Byproduct recovery by ultrafiltration"**

(73) Proprietor: **PFIZER INC.**
**235 East 42nd Street**
**New York, N.Y. 10017 (US)**

(72) Inventor: **Ho, Lewis**
**58 Inchcliffe Drive Gales Ferry**
**New London Connecticut (US)**
Inventor: **Taylor, Roy Jasper**
**27 Dunbar Road Quaker Hill**
**New London Connecticut (US)**

(74) Representative: **Moore, James William, Dr.**
**Pfizer Limited Ramsgate Road**
**Sandwich Kent CT13 9NJ (GB)**

(56) References cited:
**INDUSTRIAL AND ENGINEERING CHEMISTRY FUNDAMENTALS, vol. 10, no. 1, February 1971, pages 113-120, Washington, D.C., USA, R.L. GOLDSMITH: "Macromolecular ultrafiltration with microporous membranes"**

Courier Press, Leamington Spa, England.

**0 069 523**

## Description

Ultrafiltration is the term applied to the separation of relatively high-molecular weight solutes (*e.g.,* proteins, natural gums, polymers, other complex organic compounds) and colloidally dispersed substances such as clays, pigments, minerals, latex particles, microorganisms, etc. from their solvents (usually water). In these systems, the osmotic pressure of the solute is usually negligible and plays no important role in the separation process.

Membrane ultrafiltration, the hydraulic pressure activated separation of solutions into their individual components by passage through synthetic semi-permeable membranes, constitutes a continuous separation process which does not involve a phase change or interphase mass transfer. The process utilizes specially structured and constituted polymeric films as molecular screens which discriminate between solute and solvent molecules on a basis of differences in molecular size, shape or chemical structure.

Representative of the many and extensive reviews of ultrafilter membranes and ultrafiltrations are those of Ferry, J. D., Chemical Reviews, *18*, 373—455 (1937) and Michaels, A. S., Chemical Engineering Progress, *64*, No. 12, 31—43 (1968).

Operationally, membrane ultrafiltration is a simple process. A feed solution under pressure flows over the surface of a suitably supported membrane, and solvent and certain solute species pass through the membrane and are collected as ultrafiltrate (permeate). Other solute species and suspended matter are retained by the membrane and a concentrated solution or suspension of these species, the retentate, is recovered from the upstream chamber or feed side of the membrane. Hence, by proper membrane selection, it is possible to concentrate, purify and fractionally separate many solutions, the sole energy requirement being that required to pump the feed liquid through the equipment at the desired pressure.

Ultrafiltration as a separation technique has found wide industrial application for the removal and/or recovery of solutes from liquids by physical means, both in quantity and at low cost. There are only a few reports or accounts of the demonstration of membrane ultrafiltration for the separation or concentration of biopolymers in aqueous solution. These high molecular weight materials form true, single-phase solutions in water. In most of these reports the primary objective was to illustrate the membrane retention characteristics using high molecular weight materials but in low viscosity solutions. None of these reports have dealt with highly viscous biopolymers (viscosity of greater than 3000 centipoise) because up to the present time this has seemed an impossibility and in fact such separation or concentration is impossible unless the material in question is of a *highly pseudoplastic* nature.

U.S. Patent 3,541,006 claims the introduction of inert particles to improve flux in a polysaccharide solution in batch-stirred vessels and ultra-filtration of batch cells. U.S. Patent 3,567,810 claims a process for forming a polymeric anisotropic membrane. A 1% dextran solution was used to illustrate the rejectivity of the membrane. U.S. Patents 2,128,551 and 3,856,569 are both concerned with recovering alginous material from seaweed. Both the '551 and the '569 patents use ultrafiltration for concentration but they deal with materials of low viscosity and not having pseudoplastic behavior. Furthermore, both deal with seaweed-derived materials and not with microbial-derived biopolymers.

Of emerging and growing interest are the highly pseudoplastic xanthan biopolymers. These biopolymers are produced by the cultivation in suitable aqueous nutrient media of microorganisms belonging to the genus *Xanthomonas*.

The hydrophillic colloids produced by *Xanthomonas* species are polysaccharides which contain mannose, glucose, glucuronic acid, O-acetyl radicals and ketal-linked pyruvic acid. These gums and their derivatives have found wide food and industrial applications. Of special interest is the increasing focus on the use of *Xanthomonas* gums in displacement of oil from partially depleted reservoirs.

Typically, oil is recovered from underground reservoirs via a series of sequential operations. A new well will generally produce a limited amount of oil as a result of release of internal pressure in the well. As this pressure becomes depleted, it is necessary to pump further quantities of oil by mechanical means. These measures recover only about 25% of the total oil stored in the reservoir. A great deal of oil is still trapped within the pores of the formation. Further enhancement of recovery can then be effected by secondary recovery. In one method of recovery, a waterflood is carried out by pumping water into a well or series of wells, displacing part of the trapped oil from surrounding wells. However, waterflooding still leaves about 55—60% of the available oil trapped in the formation. The explanation for this phenomenon is that the water has a very low viscosity compared to the crude oil and tends to follow the path of least resistance, fingering through the oil and leaving large pockets untouched. In addition, surface forces in the formation tend to bind the oil and prevent its displacement.

A number of processes have been developed in recent years to recover further quantities of oil from these reservoirs by the use of mobility control solutions which enhance oil displacement by increasing the viscosity or permeability of the displacing fluid. Of interest are those enhanced recovery processes employing flooding with a polysaccharide or polyacrylamide to increase the viscosity of the displacing fluid. Variations of this process include the use of surfactants and cosurfactants to release the oil from the rock formation. Polyacrylamides have been found to suffer such deficiencies as viscosity loss in brines and severe shear sensitivity. Since, as was well documented in the prior art, xanthan gum is relatively insensitive to salts (does not precipitate or lose viscosity under normal conditions), is shear stable,

2

thermostable and viscosity stable over a wide pH range, xanthan gum is a good displacing agent. Moreover, the gum is poorly adsorbed on the elements of the porous rock formations and it gives viscosities useful in enhanced oil recovery (5 to 90 centipoise units (5 · 10⁻³ to 90 10⁻³ Pa · s) at 1.32 sec.⁻¹ shear rate) at low concentrations (100 to 3000 ppm). The use of solutions of xanthan gum or derivatives of xanthan gum for oil recovery is described in U.S. Patents 3,243,000; 3,198,268; 3,532,166; 3,305,016; 3,251,417; 3,319,715; 3,373,810; 3,434,542 and 3,729,460. It is suggested in U.S. Patent 3,305,016 that aqueous solutions containing heteropolysaccharide in sufficient quantity to increase the viscosity be employed as the thickening agent in preparing viscous waterflooding solutions. The polysaccharide may be prepared, separated, purified and then added. Alternatively, according to this reference, the entire culture after adding a bactericide (*e.g.*, formaldehyde) to kill the bacteria, may be added to the flood water.

This invention concerns a method of concentrating an aqueous, highly-pseudoplastic biopolymer solution or preconcentrate having an apparent viscosity of at least about 3,000 centipoise (3 Pa · s) comprising subjecting said solution to membrane ultrafiltration at a linear flow velocity of at least about 40 cm/s across said membrane surface while exerting an average pressure differential across said membrane of at least 2.0 atmospheres (2 10⁵ Pa). Note that the desired minimum linear flow velocity across the membrane can be achieved by a combination of pumping rate and/or flow velocity promoters contained within the ultrafiltration device. The method is preferred in which the solution is a *Xanthomonas* biopolymer and especially a *Xanthomonas* fermentation broth, most especially that derived from *Xanthomonas campestris*.

The method is also preferred in which the activity of solution is concentrated at least 2-fold. Especially preferred is the method in which the apparent viscosity of the solution rises to at least about 10,000 centipoise (10 Pa · s). The preferred temperature for the method is from about 20°—50°C.

There is an increasing trend toward the use of *Xanthomonas* fermentation broths for use in enhanced oil recovery. However, the transportation of these fermentation broths from the fermentation plants to widely dispersed oil fields involves considerable freight costs because of the tremendous volumes of broths involved. In order to reduce these volumes and shipping costs, efforts have been directed toward volume reduction by evaporation under vacuum or by precipitation of the xanthans by addition of large volumes of water-miscible solvents. These methods, unfortunately, add to process costs and may alter physical properties of the xanthan biopolymer.

It has been found, surprisingly, that highly pseudoplastic biopolymer solutions including gelatinous and viscous *Xanthomonas* fermentation broth can be readily and economically concentrated by the ultrafiltration process. In the case of *Xanthomonas* broths, such concentration is achieved without altering xanthan activity or affecting the injectivity of the reconstituted solution. The process is adaptable to the concentration of viscous *Xanthomonas* solutions and preconcentrate, including undiluted and diluted whole or filtered *Xanthomonas* fermentation broths. Another important feature of the ultrafiltration process is that the biopolymer solutions can be concentrated without altering the polymer structure and other physical properties of the solutions.

Pseudoplastic materials are non-Newtonian fluids in which viscosity decreases with increasing shear rate. Some materials, such as xanthan, can be termed "highly pseudoplastic"; i.e., their viscosity decreases drastically with a relatively small increase in shear rate. Other pseudoplastic materials, such as pullulan biopolymer, have less pseudoplastic behavior in that their viscosity decrease is not as pronounced as with xanthan. Thus, under identical conditions of ultrafiltration, pullulans can only achieve about one-tenth of the flux rate of xanthan and they can only be concentrated to a viscosity of below about 3000 centipoise (3 Pa · s).

The novel ultrafiltration application of this invention is practical for concentrating viscous broths and preconcentrates up to 20% xanthan concentration, which has a viscosity of greater than 20,000 centipoise (20 Pa · s) and even higher, while at the same time retaining the xanthan activity, injectibility and other important physical properties of *Xanthomonas* fermentation broths for use in enhanced oil recovery. The starting materials may be of a wide range of viscosities depending on the biopolymer and its concentration. In general, a *Xanthomonas* fermentation broth at about 3% concentration will have an apparent viscosity of 3000—6000 centipoise (3—6 Pa · s) by definition. The present invention is concerned with a method of concentrating in this *uncommonly* high viscosity range. Apparent viscosity is defined as the viscosity measured by the Brookfield Synchro-Lectric viscometer with LV4 spindle at 30 rpm and 25°C. The process is carried out at a linear flow velocity of at least 40 cm/sec across the membrane surface and preferably at between 50 and 200 cm/s. The process is operated at an average pressure differential of at least 2.0 atmospheres, preferably between 4 and 14 atmospheres (4—14 10⁵ Pa). Average pressure differential is defined as the numerical average of the difference in pressure across the membrane as measured at the inlet and outlet sections of the ultrafiltration device. Linear velocity across the membrane is defined as the linear velocity which the ultrafiltration membrane "sees", and is dependent upon pumping rate and internal baffling within the UF device.

The ultrafiltration equipment is preferably selected from any of those specially equipped to operate with relatively large flow channels in either turbulent or laminar flow and under sufficiently high pressure and linear flow velocities for achieving reasonable flux rate. Examples would include the tubular configuration manufactured by Western Dynetics, Inc. (Newbury Park, Ca) and the spiral wound configuration manufactured by Abcor, Inc. (Wilmington, Mass.). While the process may be satisfactorily

**0 069 523**

operated at temperatures between 10 and 60°C., it is preferably operated between about 20°—50°C. so that the physical properties of the retentate are substantially unaltered.

The ultrafiltration method may be applied to the concentration of any pseudoplastic aqueous solution. In the special case of xanthan solutions, the fermentation broth may be any of those produced employing standard production media. The preferred *Xanthomonas* fermentation broths are those described in U.S. Patent 4,119,546. These *Xanthomonas* fermentation broths are substantially free of insoluble matter having a particle size greater than about 3 μm. Broths derived by fermenting *Xanthomonas campestris* are especially preferred. Mobility control solutions (100—3000 ppm xanthan) prepared from these whole fermentation broths can be injected into subterranean oil reservoirs without the need for prior filtration. Ultrafiltration provides a simple and economical process for concentrating these fermentation broths. The highly viscous retentates can be economically shipped to oil field sites for subsequent dilution to mobility control solutions with unaltered injectability for use in enhanced oil recovery. This concentration process can be also applied to reconcentrating xanthan solutions which have been diluted for heat and/or other treatment.

The active *Xanthomonas* polymer concentration is defined as that weight percent content of xanthan which could yield a final concentration of 500 ppm activity, if diluted to 10 centipoise (under 6 rpm, 25°C, Brookfield synchron viscometer) with a 500 ppm salt solution (NaCl:CaCl$_2$=10:1).

Injectability is an important property of mobility control solutions. It is correlated with a Millipore test which is an experimental procedure that measures flow rate through a Millipore filter (0.45 to 3.0 μ pore size) as a function of volume under a constant pressure of 40 psig (276 10$^3$ Pa). The filter ratio (F.R.) is the ratio of the time to collect the fourth 250 ml of mobility control solution to the time to collect the first 250 ml of mobility control solution (see U.S. Patent 4,119,546). A filter ratio of 1.0 indicates that the solution has no plugging tendencies.

Example 1

Whole *Xanthomonas* fermentation broth (2.82% xanthan) was concentrated about three-fold in a laboratory ultrafiltration unit (Model 4 SFPP manufactured by Western Dynetics, Inc. employing a 0.37 m$^2$ polysulfone membrane (10,000 Mol. Wt. cut-off) at an average flow velocity of 122 cm/sec. and pressure differential of 7—10 atm (7—10 10$^5$ Pa). and a temperature of 25°C.

| Time (hrs) | Retentate vol (liters) | Flux rate (lsmd)* | Active xanthan conc. (%) | Apparent visc. (cps) | Xanthan content (kg) | F.R. |
|---|---|---|---|---|---|---|
| 0 | 14.74 | 442 | 2.82 | 4200[(1)] | 0.42 | 1.53 |
| 30 | 11.35 | 435 | 3.7 | | 0.42 | |
| 50 | 9.2 | 407 | 4.6 | | 0.42 | |
| 70 | 7.43 | 407 | · 5.6 | | 0.42 | |
| 100 | 5.26 | 406 | 7.8 | 8200[(1)] | 0.41 | 1.54 |

* lsmd=liter per square meter per 24 hour day.
[(1)] 1cp=10$^{-3}$ Pa · s

Example 2

Whole *Xanthomonas* fermentation broth (3.15% xanthan) was concentrated about three-fold at an average temperature of 30—36°C and pressure of 18.5—20 atm (18.5—20 10$^5$ Pa). and flow velocity of 122 cm/s by the ultrafiltration method of Example 1.

4

**0 069 523**

| Time (hrs) | Retentate vol. (liters) | Flux rate (lsmd) | Apparent visc. (cps)[1] | Active xanthan conc. (%) | Xanthan contents (kg) | F.R. |
|---|---|---|---|---|---|---|
| 0 | 160 | 697 | 5100 | 3.15 | 5.04 | 1.34 |
| 2 | 136 | 737 | 5700 | 3.8 | 5.2 | — |
| 3 | 126 | 725 | — | — | — | — |
| 4 | 116 | 689 | 6100 | 4.3 | 5.0 | — |
| 5 | 106 | 640 | — | — | — | — |
| 6 | 96 | 627 | — | — | — | — |
| 8 | 77.2 | 587 | — | — | — | — |
| 10 | 67 | 485 | 9000 | 8.3 | 5.56 | 1.40 |
| 12 | 59.2 | 407 | 11000 | 9.6 | 5.68 | 1.37 |
| 13 | 54 | 310 | 13300 | 11.5 | 5.72 | 1.38 |

[1] $1cp = 10^{-3}$ Pa · s

Example 3

Whole *Xanthomonas* fermentation broth of an apparent viscosity of 4800 (4.8 Pa · s) centipoise units (4% xanthan) was concentrated to an apparent viscosity of greater than 20,000 (20 Pa · s) (estimated 50,000 (50 Pa · s)) centipoise (to 22% xanthan) at a temperature of 25—35°C at a pressure of 8—14 atmospheres (8—14 $10^5$ Pa) and at an average linear flow velocity of 190 cm/s by the ultrafiltration method of Example 1, using a larger (1—2 m²) model of the Western Dynetics' ultrafilter described in Example 1.

Example 4

Whole fermentation broth produced from yet a different strain of *Xanthomonas campestris* having an initial concentration of 2.5% xanthan and an initial viscosity of 8100 cps (8.1 Pa · s) was concentrated five—six fold to a terminal concentration of 13.3% xanthan and greater than 20,000 cps (20 Pa · s) (estimated viscosity 80,000 (80 Pa · s)) at a temperature of 25—35°C., at a pressure of 8—14 atmospheres (8—14×$10^5$ Pa) and at an average linear flow velocity of 190 cm/s by the ultrafiltration method of Example 3.

Example 5

Whole fermentation broth was produced as in Examples 1—3 above and was diluted and heat-treated to a viscosity of 450 centipoise (0.45 Pa · s). The solution was then partially concentrated using a common ultrafiltration system (Romicon HFMI.1-43-PM50 2 cartridge unit) to 3% with a viscosity of 3600 centipoise (3.6 Pa · s) at a temperature of 25°C, an average pressure differential of 2—2.5 atmospheres (2—2.5×$10^5$ Pa) and an average linear viscosity of 210 cm/sec. The preconcentration process was terminated as the flux rate decreased from 2500 to 200 lpmd while the inlet pressure reached the maximum pressure allowable for the membrane system (3 atmospheres) (3 $10^5$ Pa).

This preconcentrate can be further concentrated by the method of Example 1 to a concentration of about 9% or above at a viscosity of about 10000 centipoise (10 Pa · s) or above.

Example 6

Whole fermentation broth produced as in Examples 1—3, having an initial concentration of 3.4% xanthan, and an initial viscosity of about 5,000 cps, (5 Pa · s) was concentrated to a terminal concentration of 9.4% xanthan and about 10,000 cps (10 Pa · s) viscosity in a spiral wound ultrafiltration device (manufactured by Abcor, Inc.), employing 5 ft² of a nominal 20,000 molecular weight cut-off membrane, at a temperature of 25°—30°C, an average pressure differential of 4—5 atmospheres (4—5×$10^5$ Pa), and an average cross flow velocity (based on flow rate and internal turbulance generators) of approximately 50 cm/s.

**Claims**

1. A method of concentrating an aqueous, highly pseudoplastic biopolymer solution or preconcentrate having an apparent viscosity of at least 3000 centipoise (3 Pa · s) comprising subjecting said solution or

5

preconcentrate to membrane ultrafiltration at a linear flow velocity of at least about 40 cm/s across said membrane surface while exerting an average pressure differential across said membrane of at least about 2.0 atmospheres (2 10$^5$ Pa).

2. The method of claim 1 in which said aqueous, highly pseudoplastic solution is a *Xanthomonas* biopolymer.

3. The method of claims 1 or 2 in which the apparent viscosity of said solution is increased to at least 10,000 centipoise (10 Pa · s).

4. The method of claims 1 or 2 in which said solution is a fermentation broth.

5. The method of claim 4 in which said broth is *Xanthomonas campestris* fermentation broth.

6. The method of claims 1 or 2 in which the activity of said solution is concentrated at least 2-fold.

7. The method of claims 1 or 2 in which said solution is maintained at a temperature of from about 20°—50°C.

### Patentansprüche

1. Verfahren zum Konzentrieren einer wäßrigen, in hohem Maße pseudoplastischen Biopolymerlösung oder eines Vorkonzentrates mit einer scheinbaren Viskosität von mindestens 3000 Centipoise (3 Pa · s), das die Durchführung einer Membranultrafiltration bei der Lösung oder dem Vorkonzentrat bei einer linearen Fließgeschwindigkeit von mindestens etwa 40 cm/s über die Membranoberfläche, während ein durchschnittlicher differentieller Druck durch die Membran von mindestens etwa 2,0 Atmosphären, (2 · 10$^5$ Pa) ausgeübt wird, umfaßt.

2. Verfahren nach Anspruch 1, worin die wäßrige in hohem Maße pseudoplastische Lösung ein *Xanthomonas* Biopolymer ist.

3. Verfahren nach Anspruch 1 oder 2, worin die scheinbare Viskosität der Lösung auf mindestens 10.000 Centipoise (10 Pa · s) erhöht ist.

4. Verfahren nach Anspruch 1 oder 2, worin die Lösung eine Fermentationsbrühe ist.

5. Verfahren nach Anspruch 4, worin diese Brühe eine Fermentationsbrühe von *Xanthomonas campestris* ist.

6. Verfahren nach Anspruch 1 oder 2, worin die Aktivität der Lösung mindenstens zweifach konzentriert wird.

7. Verfahren nach Anspruch 1 oder 2, worin die Lösung bei einer Temperatur von etwa 20° bis 50°C gehalten wird.

### Revendications

1. Procédé pour concentrer une solution aqueuse ou un préconcentré aqueux de biopolymère hautement pseudoplastique ayant une viscosité apparente d'au moins 3000 centipoises (3 Pa · s), consistant à soumettre ladite solution ou ledit préconcentré à une ultrafiltration à travers une membrane à une vitesse d'écoulement linéaire d'au moins environ 40 cm/s à travers la surface de ladite membrane tout en exerçant une différence de pression moyenne de part et d'autre de la membrane d'au moins environ 2,0 atmosphères (2 · 10$^5$ Pa).

2. Procédé suivant la revendication 1, dans lequel ladite solution aqueuse hautement pseudoplastique est un biopolymère dérivé de *Xanthomonas*.

3. Procédé suivant la revendication 1 ou 2, dans lequel la viscosité apparente de ladite solution est élevée à au moins 10 000 centipoises (10 Pa · s).

4. Procédé suivant la revendication 1 ou 2, dans lequel ladite solution est un bouillon de fermentation.

5. Procédé suivant la revendication 4, dans lequel ledit bouillon est un bouillon de fermentation de *Xanthomonas campestris*.

6. Procédé suivant la revendication 1 ou 2, dans lequel l'activité de ladite solution est concentrée d'un facteur 2 au moins.

7. Procédé suivant la revendication 1 ou 2, dans lequel ladite solution est maintenue à une température d'environ 20 à 50°C.